(19) Europäisches Patentamt
European Patent Office
Office européen des brevets

(11) Veröffentlichungsnummer: **0 220 519**
**A2**

(12) # EUROPÄISCHE PATENTANMELDUNG

(21) Anmeldenummer: **86113417.9**

(51) Int. Cl.[4]: **A61K 31/50**, A61K 31/55

(22) Anmeldetag: **30.09.86**

(30) Priorität: **02.10.85 DE 3535170**

(43) Veröffentlichungstag der Anmeldung:
**06.05.87 Patentblatt 87/19**

(84) Benannte Vertragsstaaten:
**AT BE CH DE FR GB IT LI NL SE**

(71) Anmelder: **BASF Aktiengesellschaft**
**Carl-Bosch-Strasse 38**
**D-6700 Ludwigshafen(DE)**

(72) Erfinder: **Geiss, Karl-Heinz, Dr.**
**Kirchenstrasse 8**
**D-6711 Beindersheim(DE)**
Erfinder: **Rossy, Phillip A., Dr.**
**39 Forest Drive**
**Hilldale, N.J. 07642(US)**
Erfinder: **Thyes, Marco, Dr.**
**Thorwaldsenstrasse 1**
**D-6700 Ludwigshafen(DE)**
Erfinder: **Koenig, Horst, Prof. Dr.**
**Pariser Strasse 4**
**D-6700 Ludwigshafen(DE)**
Erfinder: **Lehmann, Hans Dieter, Prof. Dr.**
**Im Hefen 15**
**D-6945 Hirschberg(DE)**
Erfinder: **Traut, Martin, Dr.**
**Muehltalstrasse 125**
**D-6900 Heidelberg(DE)**
Erfinder: **Gries, Josef, Dr.**
**Roemerweg 43**
**D-6706 Wachenheim(DE)**

(54) **Verwendung eines 2-Aryl-3,4-diaza-bicyclo[4.n.O]alken-(2)-ons-(5) zur Herstellung eines Mittels zur Behandlung der Herzinsuffizienz.**

(57) Die vorliegende Erfindung betrifft die Verwendung von 2-Aryl-3,4-diaza-bicyclo-[4.n.0]alken-(2)-onen-(5) der Formel

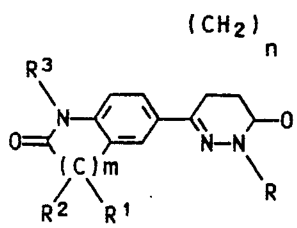

I

worin m und n, R, R', R² und R³ die angegebene Bedeutung besitzen, zur Behandlung der Herzinsuffizienz.

EP 0 220 519 A2

## Verwendung eines 2-Aryl-3,4-diaza-bicyclo[4.n.0]alken-(2)-ons-(5) zur Herstellung eines Mittels zur Behandlung der Herzinsuffizienz

Die Erfindung betrifft die Verwendung von 2-Aryl-3,4-diaza-bicyclo[4.1.0]hepten-(2)-onen-(5), 2-Aryl-3,4-diazabicyclo[4.2.0]-octen-(2)-onen-(5) und 2-Aryl-3,4-diaza-bicyclo[4.3.0]-nonen-(2)-onen-(5) bei der Herstellung eines Mittels zur Therapie der Herzinsuffizienz.

In der europäischen Patentschrift 68 310 werden Diaza-bicyclo[4.n.0]alkenone der allgemeinen Formel I

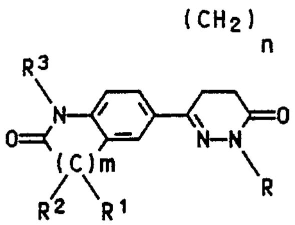

I

beschrieben, worin

m und n gleich oder verschieden sind und die Zahlen 1, 2 oder 3 bedeuten, und

R, $R^1$, $R^2$ und $R^3$ gleich oder verschieden sind und Wasserstoffatome oder Akylgruppen mit 1 bis 6 C-Atomen bedeuten, wobei $R^1$ und $R^2$ am gleichen oder an verschiedenen C-Atomen sitzen können, die starke thrombocytenaggregationshemmende und blutdrucksenkende Wirkungen besitzen.

In der EP-A-155 798 werden monocyclische Dihydropyridazinone, die denen der vorliegenden Formel I ähnlich sind, aber keinen Diaza-bicyclus enthalten, mit positiv inotropen Wirkungen beschrieben.

Es wurde nun gefunden, daß die Verbindungen der Formel I stark cardiotone, d.h. positiv inotrope und coronardilatatorische Wirkungen besitzen. Außerdem hemmen sie die low $k_m$-cAmp-Phospho-diesterase. Die Verbindungen sind demnach als Cardiotonika zur Therapie der verschiedenen Formen der Herzinsuffizienz besonders geeignet.

Bevorzugt bedeuten

n die Zahl 1 oder 2, insbesondere 1,

R ein Wasserstoffatom,

$R^1$, $R^2$ und $R^3$ unabhängig voneinander Wasserstoffatome oder Methylgruppen,

$R^3$ insbesondere ein Wasserstoffatom.

Insbesondere sind zu nennen:

2-(2,3,4,5-Tetrahydro-benzo[b]-azepin-2-(1H)-on-7-yl)-3,4-diazabicyclo-[4.1.0]-hepten(.)-on-(5) und

2-(3,3-Trimethylindolin-2-on-5-yl)-3,4-diaza-bicyclo-[4.1.0]hepten-(2)-on-(5).

Die Verbindungen der Formel I werden hergestellt, indem man eine Cycloalkancarbonsäure der Formel II

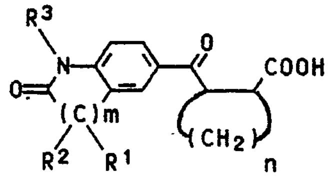

II,

in der $R^1$, $R^2$ und $R^3$ die oben angegebenen Bedeutungen haben, mit einem Hydrazin der Formel $NH_2NHR$, in der R die oben angegebene Bedeutung hat, umsetzt.

Die Cyclisierung einer Verbindung der Formel II mit einem Hydrazin der Formel $NH_2NHR$, in der R die oben angegebenen Bedeutungen hat, zu einem Diaza-bicyclo[4.n.0]alkenon der Formel I erfolgt vorteilhaft in einem unter den Reaktionsbedingungen inerten Lösungsmittel, insbesondere einem niederen Alkohol wie Methanol, Ethanol oder Propanol, einem cyclischen aliphatischen Ether wie Tetrahydrofuran oder Dioxan, oder einem Dialkylformamid, wie Dimethylformamid, und bei Temperaturen von 40 bis 150°C, vorzugsweise 60 bis 120°C. In der Regel werden hierbei je 1 Mol der Verbindung der Formel II 1 bis 1,2 Mol des Hydrazins verwendet.

Die Ausgangsverbindungen der Formel II werden durch Umsetzung einer Verbindung der Formel III

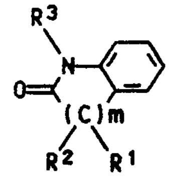

III,

in der $R^1$, $R^2$, $R^3$ und m die oben angegebenen Bedeutungen haben, mit 1,2-Cyclopropan-, 1,2-Cyclobutan- oder 1,2-Cyclopentan-dicarbonsäureanhydrid in Gegenwart von Aluminiumchlorid unter den Bedingungen einer Friedel-Crafts-Acylierung erhalten.

Diese Acylierung kann in einem Lösungsmittel, beispielsweise Schwefelkohlenstoff, bei Temperaturen von 25 bis 150°C durchgeführt werden. Sie kann auch in einer Dimethylformamid/Aluminiumchlorid-Schmelze bei Tempe raturen zwischen 50 und 200°C, vorzugsweise 100 bis 160°C, erfolgen. Dabei ist es zweckmäßig, auf 1 Mol 1,2-Cyclopropan-, 1,2-Cyclobutan-oder 1,2-Cyclopentan-dicarbonsäureanhydrid bzw. 1 Mol einer Verbindung der Formel II, etwa 10 Mol Aluminiumchlorid und etwa 2,5 Mol Dimethylformamid zu verwenden.

Es sei darauf hingewiesen, daß die erfindungsgemäß zu verwendenden Verbindungen der Formel I in den Stellungen 1 und 6 des 3,4-Diaza-bicycloalkenon-Ringes asymmetrische Kohlenstoffatome aufweisen und in der 3-Position des Indolinons bzw. der 5-oder 4-Position des Tetrahydrochinolinon-bzw. der 3-, 4- oder 5-Position des Tetrahydrobenzo[b]azepinonringes asymmetrische Kohlenstoffatome aufweisen können. Die Erfindung umfaßt alle Enantiomeren und diastereomeren Formen oder Verbindungen der Formel I.

Zur Untersuchung der pharmakodynamischen Eigenschaften der erfindungsgemäßen Verbindungen wurde die folgende Methode verwendet:

Die positiv inotrope Wirkung wurde an 2 bis 4 narkotisierten (200 bis 330 mg/kg Hexobarbital-Natrium intramuskulär) Kätzen (2,4 bis 4,5 kg) nachgewiesen. Sie wurde als Zunahme der maximalen Druckanstiegsgeschwindigkeit $(dp/dt)_{max}$ mit Tip-Manometern aus dem Druckverlauf in der linken Herzkammer und mit Differenzierverstärkern (Fa. Hellige, Freiburg) ermittelt. Die Applikation erfolgte intravenös in eine V. brachialis oder intraduodenal. Das Applikationsvolumen betrug 0,5 ml/kg i.v. bzw. 1,0 mg/kg i.d. appliziert.

Methode zur Untersuchung der Hemmung der low-$k_m$-cAmp-Phosphodiesterase:

Die enzymatische Bestimmung erfolgt mit dem 100.000 x g Überstand vom Homogenat humaner Thrombozyten nach der Vorschrift von Asano et al [Asano, T., Ochiai, Y. and Midaka, H., Mol. Pharmacol., 13, 400-406 (1977)] mit $^3H$-markiertem cAmp und cGMP in jeweils 2 Konzentrationen als Substrat:

$$cAmp \xrightarrow{PDE} 5'\text{-}Amp \xrightarrow{5'\text{-}Nucleotidase} Adenosin + Pi$$

$$cGMP \longrightarrow 5'\text{-}GMP \longrightarrow Guanosin + Pi$$

Die Nucleoside werden an Kationenaustauschern vom Ausgangsmaterial getrennt und durch $^3H$-Messung im Eluat quantifiziert.

Die zu verwendenden Verbindungen können in üblicher Weise oral oder parenteral (intravenös, intramuskulär, intraperitoneal) verabfolgt werden. Die Dosierung hängt vom Alter, Zustand und Gewicht des Patienten sowie von der Applikationsart ab. In der Regel beträgt die tägliche Wirkstoffdosis zwischen etwa 0,1 und 10 mg/kg Körpergewicht bei oraler Gabe und zwischen etwa 0,01 und 1,0 mg/kg Körpergewicht bei

parenteraler Gabe. Im Normalfall werden mit täglichen Dosen von 0,5 bis 5 mg/kg oral und 0,05 bis 0,5 mg/kg parenteral zufriedenstellende Ergebnisse erzielt.

Die Verbindungen können in den gebräuchlichen galenischen Applikationsformen fest oder flüssig angewendet werden, z.B. als Tabletten, Filmtabletten, Kapseln, Pulver, Granulate, Dragees oder Suppositorien. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Emulgatoren, Lösungsmitteln, Retardierungsmitteln, Antioxidantien und/oder Treibgasen verarbeitet werden (vgl. H. Sucker/Pharmazeutische Technologie, Thieme-Verlag, Stuttgart, 1978). Die so erhaltenen Pharmaka enthalten den Wirkstoff normalerweise in einer Konzentration von 1 bis 99 Gew.%.

Herstellung der Ausgangsverbindungen

a) Zu 120 g (0,9 mol) wasserfreiem Aluminiumchlorid gibt man unter Rühren innerhalb weniger Minuten tropfenweise 20 ml (0,26 mol) Dimethylformamid, wobei eine stark exotherme Reaktion eintritt. Man fügt dann bei 140°C portionsweise ein Gemisch aus 11,8 g (0,089 mol) Indolinon-2 und 11,3 g (0,09 mol) Cyclobutandicarbonsäureanhydrid zu und rührt anschließend noch 10 min bei 140°C nach. Die Schmelze wird nun in 0,5 kg Eis eingetragen. Der ausgefallene Festkörper wird abgesaugt und die Wasserphase mehrmals mit Essigester extrahiert. Man erhält 21,7 g (93,6 %)

cis-2(Indolin-2-on-5-oyl)cyclobutancarbonsäure, Fp. = 211 bis 212°C.

Analog erhält man:

b) cis-2-(1-Methyl-indolin-2-on-5-oyl)cyclobutancarbonsäure, Fp. 210 bis 212°C, Ausbeute 84 %.

c) cis-2-(1-Indolin-2-on-5-oyl)cyclopropancarbonsäure, Fp. 187 bis 190°C, Ausbeute 81 %.

d) cis-2-(1-Methylindolin-2-on-5-oyl)cyclopropancarbonsäure, Fp. 237 bis 240°C, Ausbeute 81,5 %.

e) cis-2-(1,2,3,4-Tetrahydrochinolin-2-on-6-oyl)cyclobutancarbonsäure, Fp. 155 bis 158°C, Ausbeute 75 %.

f) cis-2-(1,2,3,4-Tetrahydrochinolin-2-on-6-oyl)cyclopropancarbonsäure, Fp. 190 bis 194°C, Ausbeute 57 %.

g) cis-2-(2,3,4,5-Tetrahydro-benzo[b]azepin-2-(1H)-on-7-oyl)cyclopropancarbonsäure als hellgelbes Öl, Ausbeute 70 %.

Analyse für $C_{15}H_{15}NO_4$ (273):

ber. C 65,9 H 5,5 N 5,1

gef. C 65,9 H 5,4 N 4,9

h) cis-2-(3,3-Dimethyl-indolin-2-on-5-oyl)-cyclopropancarbonsäure, amorph, Ausbeute 58 %.

i) cis-2-(3,3-Dimethyl-indolin-2-on-5-oyl)-cyclobutancarbonsäure, amorph, Ausbeute 49 %.

k) cis-2-(3,3-Dimethyl-1,2,3,4-tetrahydrochinolin-2-on-6-oyl)-cyclopropancarbonsäure, amorph, Ausbeute 39 %.

l) cis-2-(3,3-Dimethyl-1,2,3,4-tetrahydrochinolin-2-on-6-oyl)-cyclobutancarbonsäure, amorph, Ausbeute 55 %.

m) cis-2-(3-Methyl-2,3,4,5-tetrahydro-benzo[b]-azepin-2-(1H)-on-7-oyl)-cyclopropancarbonsäure, amorph, Ausbeute 58 %.

<u>Beispiel 1</u>

A) 5,22 g (0,02 mol) cis-2-(Indolin-2-on-5-oyl)cyclobutancarbonsäure werden mit 1,1 g (0,022 mol) Hydrazinhydrat und 50 ml Ethanol 11 Stunden am Rückfluß gehalten. Nach dem Absaugen bei Raumtemperatur und Umkritallisieren aus Dimethylformamid/Wasser erhält man 4,7 g (92 %) 2-(Indolin-2-on-5-yl)-3,4-diaza-bicyclo[4.2.0]-octen-(2)-on-(5), Fp. 309 bis 312°C.

Analyse für $C_{14}H_{13}N_3O_2$ (255):

ber. C 65,8 H 5,1 N 16,5

gef. C 65,5 H 5,3 N 16,5

Analog erhält man:

B) 2-(1-Methylindolin-2-on-5-yl)-3,4-diaza-bicyclo[4.2.0]-octen-(2)-on-(5), Fp. 256 bis 259°C, Ausbeute 85 %.

Analyse für $C_{15}H_{15}N_3O_2$ (269):

ber. C 66,9 H 5,6 N 15,6

gef. C 66,6 H 5,7 N 15,5

C) 2-(Indolin-2-on-5-yl)-3,4-diaza-bicyclo[4.1.0]-hepten-(2)-on-(5), Fp. 305 bis 307°C, Ausbeute 81 %.

Analyse für $C_{13}H_{11}N_3O_2$ (241):

ber. C 64,7 H 4,6 N 17,4

gef. C 64,7 H 4,6 N 17,5

D) 2-(1-Methylindolin-2-on-5-yl)-3,4-diaza-bicyclo[4.1.0]-hepten-(2)-on-(5), Fp. 265 bis 267°C, Ausbeute 51 %.

Analyse für $C_{14}H_{13}N_3O_2$ (255):

ber. C 65,9 H 5,1 N 16,5

gef. C 65,4 H 5,1 N 17,0

E) 2-(1,2,3,4-Tetrahyrochinolin-2-on-6-yl)-3,4-diaza-bicyclo[4.2.0]-octen-(2)-on-(5), Fp. 349 bis 353°C, Ausbeute 84 %.

Analyse für $C_{15}H_{15}N_3O_2$ (269):

ber. C 66,9 H 5,6 N 15,6

gef. C 66,7 H 5,6 N 15,5

F) 2-(1,2,3,4-Tetrahydrochinolin-2-on-6-yl)-3,4-diaza-bicyclo-[4.1.0]-hepten-(2)-on-(5), Fp. 310 bis 311°C (Zers.), Ausbeute 75 %.

Analyse für $C_{14}H_{13}N_3O_2 \times 1/4\ H_2O$:

ber. C 64,8 H 5,2 N 16,4

gef. C 64,7 H 5,2 N 16,2

G) 2-(1,2,3,4-Tetrahydrochinolin-2-on-6-yl)-4-methyl-3,4-diaza-bicyclo-[4.1.0]-hepten-(2)-on-(5), Fp. 255 bis 259°C, Ausbeute 76 %.

Analyse für $C_{15}H_{15}N_3O_2$ (269):

ber. C 66,9 H 5,6 N 15,6

gef. C 66,6 H 5,7 N 15,5

H) 2-(2,3,4,5-Tetrahydro-benzo[b]azepin-2-(1H)-on-7-yl)-3,4-diaza-bicyclo[4.1.0]-hepten-(2)-on-(5), Fp. 308 bis 310°C (Zers.), Ausbeute 75 %.

Analyse für $C_{15}H_{15}N_3O_2$ (269):

ber. C 66,9 H 5,6 N 15,6

gef. C 66,2 H 5,3 N 15,4

I) 2-(3,3-Dimethylindolin-2-on-5-yl)-3,4-diazabicyclo-[4.1.0]-hept-2-en-5-on, Fp. 334-335°C, Ausbeute 71 %.

Analyse für $C_{15}H_{15}N_3O_2$ (269)

ber. C 66,9 H 5,6 N 15,6

gef. C 66,7 H 5,7 N 15,5

K) 2-(3,3-Dimethylindolin-2-on-5-yl)-3,4-diazabicyclo-[4.2.0]-oct-2-en-5-on, Fp. 315-3317C, Ausbeute 55 %.

Analyse für $C_{16}H_{15}N_3O_2$• 0,1 $H_2O$

ber. C 67,39 H 6,08 N 14,74

gef. C 67,0 H 6,1 N 14,7

L) 2-(3,3-Dimethyl-1,2,3,4-tetrahydrochinolin-2-on-6-yl)-3,4-diazabicyclo[4.1.0]-hept-2-en-5-on, Fp. 275 bis 277°C, Ausbeute 62 %.

Analyse für $C_{16}H_{17}N_3O_2$•0,1 $H_2O$

ber. C 67,39 H 6,08 N 14,74

gef. C 67,0 H 6,2 N 14,9

M) 2-(3,3-Dimethyl1,2,3,4-tetrahydro-chinolin-2-on-6-yl)-3,4-diazabicyclo-[4.2.0]-oct-2-en-5-on, Fp. 301 bis 302°C, Ausbeute 81 %.

N) 2-(3-Methyl-2,3,4,5-tetrahydro-benzo[b]-zepin-2-(1H)-on-7-yl)-3,4-diazabicyclo[4.1.0]-hept-2-en-5-on, Fp. 305 bis 307°C, Ausbeute 21 %.

Analyse für $C_{15}H_{15}N_3O_2$ (269)

ber. C 64,8 H 5,2 N 16,4

gef. C 64,7 H 5,2 N 16,2

O) 2-(3-Methylindolin-2-on-5-yl)-3,4-diaza-bicyclo[4.1.0]-hepten-(2)-on-(5)
P) 2-(3,3-Diethylindolin-2-on-5-yl)-3,4-diaza-bicyclo[4.1.0]-hepten-(2)--on-(5)
Q) 2-(1,3,3-Trimethylindolin-2-on-5-yl)-3,4-diaza-bicyclo[4.1.0]hepten-(2)-onen-(2)-on-(5)
R) 2-(3-Methyl-1,2,3,4-tetrahydrochinolin-2-on-6-yl)-3,4-diaza-bicyclo-[4.1.0]-hepten-(2)-on-(5)
S) 2-(3-Ethyl-1,2,3,4-tetrahydrochinolin-2-on-6-yl)-3,4-diaza-bicyclo-[4.1.0]-hepten-(2)-on-(5)
T) 2-(4-Methyl-1,2,3,4-tetrahydrochinolin-2-on-6-yl)-3,4-diaza-bicyclo-[4.1.0]-nonen-(2)-on-(5)
U) 2-(3,3-Diethyl-1,2,3,4-tetrahydrochinolin-2-on-6-yl)-3,4-diaza-bicyclo[4.1.0]-hebten-(2)-on-(5)
V) 2-(1,3-Dimethyl-1,2,3,4-tetrahydrochinolin-2-on-6-yl)-3,4-diaza-bicyclo[4.1.0]-hepten-(2)-on-(5)
W) 2-(1,4-Dimethyl-1,2,3,4-tetrahydrochinolin-2-on-6-yl)-3,4-diaza-biyclo[4. 1.0]-hepten-(2)-on-(5)
X) 2-(1,3,3-Trimethyl-1,2,3,4-tetrahydrochinolin-2-on-6-yl)-3,4-diazabicyclo[4.1.0]-hepten-(2)-on-(5)

Formulierungsbeispiele

I. Es werden Tabletten folgender Zusammensetzung hergestellt:

| | |
|---|---|
| Wirkstoff | 10 mg |
| Polyvinylpyrrolidon (mittl. M.G. 25.000) | 170 mg |
| Polyethylenglykol (mittl. M.G. 4.000) | 14 mg |
| Hydroxypropylmethylcellulose | 40 mg |
| Talkum | 4 mg |
| Magnesiumstearat | 2 mg |
| | 240 mg |

Der Wirkstoff wird mit Polyvinylpyrrolidon in 10 %iger wäßriger Lösung befeuchtet, durch ein Sieb mit der lichten Maschenweite 1,0 mm getrieben und bei 50°C getrocknet. Dieses Granulat wird mit Polyethylenglykol (mittl. M.G. 4.000), Hydroxypropylmethylcellulose, Talkum und Magnesiumstearat vermischt und zu Tabletten a 240 mg verpreßt:

II. Es werden Dragees folgender Zusammensetzung hergestellt:

| | |
|---|---|
| Wirkstoff | 10 mg |
| Lactose | 90 mg |
| Maisstärke | 60 mg |
| Polyvinylpyrrolidon | 6 mg |
| Magnesiumstearat | 1 mg |
| | 167 mg |

Die Mischung der Wirkstoffsubstanz mit Lactose und Maisstärke wird mit einer 8 %igen wäßrigen Lösung des Polyvinylpyrrolidons durch ein Sieb 1,5 mm granuliert, bei 50°C getrocknet und nochmals durch Sieb 1,0 mm getrieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum besteht.

## Ansprüche

1. Verwendung von 2-Aryl-3,4-diaza-bicyclo-[4.n.0]alken-(2)-onen-(5) der Formel I

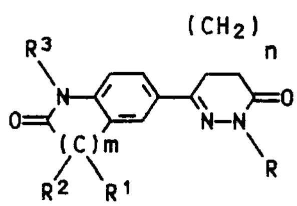

I

worin

m und n gleich oder verschieden sind und die Zahlen 1, 2 oder 3 bedeuten, und

R, $R^1$, $R^2$ und $R^3$ gleich oder verschieden sind und Wasserstoffatome oder Akylgruppen mit 1 bis 6 C-Atomen bedeuten, als Wirkstoff bei der Herstellung eines Mittels zur Bekämpfung der Herzinsuffizienz.

2. Verwendung von Verbindungen des Anspruchs 1, worin m die Zahl 1, 2 oder 3 und n die Zahl 1 oder 2 und R ein Wasserstoffatom, $R^1$, $R^2$ und $R^3$ unabhängig voneinander Wasserstoffatome oder Methylgruppen bedeuten, als Wirkstoff bei der Herstellung eines Mittels zur Bekämpfung der Herzinsuffizienz.

3. Verwendung von Verbindungen des Anspruchs 1, worin m die Zahl 1, 2 oder 3 und n die Zahl 1 bedeutet, und R und $R^3$ Wasserstoffatome, $R^1$ und $R^2$ unabhängig voneinander Wasserstoffatome oder Methylgruppen bedeuten, als Wirkstoff bei der Herstellung eines Mittels zur Bekämpfung der Herzinsuffizienz.

4. Verwendung 2-(2,3,4,5-Tetrahydro-benzo-[b]-acepin-2-(1H)-on-7-yl)-3,4-diaza-bicyclo[4.1.0]-hepten-(2)-on-(5) als Wirkstoff bei der Herstellung eines Mittels zur Bekämpfung der Herzinsuffizienz.

5. Verwendung von 2-(3,3-Dimethylindolin-2-on-5-yl)-3,4-diazabicyclo-[4.1.0]-hept-en-(2)-on-(5) als Wirkstoff bei der Herstellung eines Mittels zur Bekämpfung der Herzinsuffizienz.